# EUROPEAN PATENT SPECIFICATION

(11) **EP 1 351 679 B1**
(45) Date of publication and mention of the grant of the patent: **03.02.2010**
(21) Application number: 01991367.2
(22) Date of filing: 19.12.2001
(51) Int. Cl.: A61K 45/06, A61K 31/593, A61K 31/352, A61P 3/10

(54) **METHOD AND COMPOSITION FOR THE TREATMENT OF DIABETIC NEUROPATHY**
METHODE UND ZUSAMMENSETZUNG ZUR BEHANDLUNG DIABETISCHER NEUROPATHIE
METHODE ET COMPOSITION POUR LE TRAITEMENT DE NEUROPATHIES DIABETIQUES

(30) Priority: 21.12.2000 US 740811; 02.05.2001 US 847121
(43) Date of publication of application: 15.10.2003
(73) Proprietor: The Quigley Corporation, Doylestown, PA 18901-1349 (US)
(72) Inventor: ROSENBLOOM, Richard, A., Elkins Park, PA 19027 (US)
(74) Representative: Dunleavy, Kevin James
(86) International application number: PCT/US2001/049297
(87) International publication number: WO 2002/049575

(56) References cited:
- WO-A-00/35848
- WO-A-00/59522
- US-A- 4 250 097
- US-A- 4 591 600
- US-A- 5 948 443
- US-A- 6 103 709
- US-A- 6 103 756
- DATABASE MEDLINE [Online] TEIJIN INSTITUTE FOR BIO-MEDICAL RESEARCH (TOKYO, JAPAN) FUKUOKA M. ET AL.: 'Novel pharmacological activity of a vitamin (novel pharmacological action of vitamin D)', XP002958798 Database accession no. 98164144 & NIPPON YAKURIGAKU ZASSHI vol. 10, no. SUPPL. 1, October 1997, pages 39 - 43
- DATABASE MEDLINE [Online] DEPARTMENT OF PHARMACOGNOSY AND PHYTOCHEMISTRY, MEIJI COLLEGE OF PHARMACY (TOKYO, JAPAN) OKADA Y. ET AL.: 'Search for naturally occuring substances to prevent the complications of diabetes. II. Inhibitory effect of coumarin and flavonoid derivatives on bovine lens aldose reductase and rabbit platelet aggregation', XP002958799 Database accession no. 96047952 & CHEM. PHARM. BULL. vol. 43, no. 8, August 1995, TOKYO, pages 1385 - 1387
- DATABASE MEDLINE [Online] DEPARTMENT OF PHARMACOLOGY, MEDICAL SCIENCES, QUEEN MARY AND WESTERFIELD COLLEGE (LONDON, UK) RIAZ S. ET AL.: 'A vitamin D(3) derivative (CB1093) induces nerve growth factor and prevents neurotrophic deficits in streptozotocin-diabetic rats', XP002958800 Database accession no. 0010550414 & DIABETOLOGICA vol. 42, no. 11, November 1999, pages 1308 - 1313
- DAM VAN P S; BRAVENBOER B: "OXIDATIVE STRESS AND ANTIOXIDANT TREATMENT IN DIABETIC NEUROPATHY" NEUROSCIENCE RESEARCH COMMUNICATIONS, vol. 21, no. 1, 1997, pages 41-48, XP000870060

## Description

### BACKGROUND OF THE INVENTION

### 1. Field of the Invention

The present invention relates to a composition for the treatment of diabetic neuropathy. More particularly, the present invention relates to a composition including a combination of ingredients, which provide a surprising degree of effective relief from symptoms of diabetic neuropathy and to a methods for using the compositions to treat diabetic neuropathy, and wherein said composition is to be administered topically.

### 2. Description of the Prior Art

Diabetes mellitus is a common disease that is usually classified into insulin-dependent and non-insulin dependent types. Both types may be managed by diet, in combination with insulin in the first type and a variety of drugs in the second type. However, while conscientious patients and doctors can usually manage the changes in blood glucose associated with diabetes reasonably satisfactorily, this does not prevent long term damage to many tissues as a result of the disease. This damage may take many forms but the major types are damage to the eyes (retinopathy), nerves (neuropathy), kidneys (nephropathy) and cardiovascular system.

There are many approaches to reducing or preventing these forms of damage, which are collectively known as the long-term complications of diabetes. One approach is based on damage that results from over-production of the glucose metabolite, sorbitol, in the cells of the body. Glucose can be converted to sorbitol by the enzyme aldose reductase. High levels of sorbitol may be among the causes of diabetic complications such as diabetic neuropathy. As a result, a number of pharmaceutical companies have been developing aldose reductase inhibitors for the purpose of reducing diabetic neuropathy.

It has been established that a wide variety of flavanoids are effective inhibitors of aldose reductase, including such flavanoids as quercetin, quercitrin and myrecetrin.
However, U.S. Patent No. 4,232,040 discloses that despite the fact that these flavanoids have been shown in *in vitro* studies to be among the most potent flavanoids for aldose reductase inhibition, a need exists for aldose reductase inhibitors that can be used more effectively and in lower doses than these flavanoids.

In fact, numerous patents are devoted to the goal of developing improved aldose reductase inhibitors. Among these patents are U.S. Patent Nos. 6,069,168; 5,011,840; 4,210,667; 4,147,795; 5,866,578; and 5,561,110. Numerous other patents exist which relate to aldose reductase inhibitors.

Another approach to the treatment of diabetic neuropathy is disclosed in U.S. Patent No. 5,840,736 (Zelle et al.). In this method, pharmaceutical compositions are disclosed for stimulating the growth of neurites in nerve cells. The compositions include a neurotrophic amount of a compound and a nerve growth factor. These compositions may be administered in a number of ways including orally and topically.

Still another approach to the treatment of neuropathy is disclosed in U.S. Patent No. 5,550,249 (Della Ville et al.). In this approach, compositions suitable for treatment of vitamin H deficiencies are administered for the treatment of neuropathy. This patent relates to biotin salts with alkanolamines. The compositions may be administered orally, parenterally or topically.

U.S. Patent No. 5,665,360 (Mann) relates to the treatment of peripheral neuropathies associated with diabetes mellitus by periodic topical application of a composition containing capsicum oleoresin as the active ingredient. When applied to the skin of the affected area, pain and burning associated with the neuropathy are said to be reduced. However; capsicum oleoresin has been shown to kill nerve endings in some cases and thus this composition suffers from this disadvantage.

U.S. Patent No. 5,981,594 (Okamoto et al.) relates to a method of treatment of diabetic neuropathy using combined administration of a formulation including as an active ingredient, a prostaglandin I derivative with an anti-diabetic agent in order to improve nerve conduction velocities. Suitable anti-diabetic agents include oral hypoglycemic agents and insulin.

The Okamoto patent also contains a detailed discussion of the various types of neuropathy that may be associated with diabetes. According to this patent, nerve conduction velocity (NCV) is the most widely used method of objectively evaluating the severity of diabetic neuropathy. This patent also mentions that current methods of treating diabetic neuropathy such as dietetic therapy, administration of insulin, aldose reductase inhibitors or aminoguanidine to improve abnormal glucose metabolism, and administration of troglitazone or agents for the improvement of blood flow have been tested but found to be insufficient when a single drug was used. Also, according to this patent, methods of treatment by combined use of different therapeutic agents which have different functions had yet to be established. The patent concludes that combined drug therapies for diabetic neuropathy, aiming at recovering once reduced nerve conduction velocity, have not yet been confirmed.

U.S. Patent no. 6,103,756 (Gorsek) discloses an oral composition (col. 2,1.11) for the treatment of ocular disease such as diabetic retinopathy (col. 1,11. 50-54) comprising 400 IU of a vitamin D₃ compound, 500 IU of natural vitamin E and 100 mg of quercetin (Table 1).

Riaz, S. et al., "A vitamin D3 derivative (CB1093) induces nerve growth factor and prevents neurotrophic deficits in streptozotocin-diabetic rats," Diabetologia, 1999 Nov; 42(11): pp. 1308-1313, discloses oral treatment of streptozotocin-induced diabetic rats with 1(S), 3(R)-dihydroxy-20(R)-(1-ethoxy-5-ethyl-5-hydroxy-2-heptyn-1-yl)-9, 10-seco-pregna-5(Z), 7(E), 10 (19)-triene ("CB1093") for the purpose of preventing depletions of nerve growth factor in sciatic nerves of diabetic rats. Riaz, S. et al. concluded that, "The CB 1093 did not affect expression of nerve growth factor in dorsal root ganglia in control or diabetic rats," and that the treatment did not affect expression of nerve growth factor receptors (trkA and P76NT) in dorsal root ganglia in diabetic rats even though P75NTR expression was reduced in diabetic rats.

U.S. Patent no. 4,250,097 (Pfister) teaches that certain compounds are effective inhibitors of lens aldose reductase, as shown by *in vitro* studies. See col. 1, lines 19-42 of Pfister. Though Pfister indicates that certain aldose reductase inhibitors could potentially be used to treat diabetic neuropathy, no method of treatment of diabetic neuropathy is disclosed, nor is any evidence presented which would indicate the effectiveness of these compounds for treatment of neuropathy.

Van Dam, P.S., et al., "Oxidative Stress and Antioxidant Treatment in Diabetic Neuropathy," Neuroscience Research Communications, Vol. 31, No. 1, pp. 41-47 (1997) tests the effects of certain antioxidants on nerve blood flow (NBF) and nerve conduction velocity (NCV) in streptozotocin-diabetic rats. Van Dam concludes that, "Antioxidant treatment is beneficial in animal studies, leading to improvement of both NCV and NBF. The outcome of extensive trials should be awaited to inform us about the clinical usefulness of antioxidant treatment for the present and treatment of neuropathy in diabetic patients." See page 46, Concluding Remarks.

There remains a need in the art for an effective treatment for diabetic neuropathy that does not suffer from the disadvantage that it causes severe side effects, as do many aldose reductase inhibitors, for example.

Accordingly, it is the primary object of the present invention to provide a composition that is effective for the treatment of diabetic neuropathy.

It is another object of the present invention to provide a composition for the treatment of diabetic neuropathy that does not cause severe side effects in the patient treated with the composition.

These and other objects of the present invention will be apparent from the summary and detailed descriptions of the invention that follow.

### SUMMARY OF THE INVENTION

The present invention relates to topical composition for use in the treatment of diabetic neuropathy. The topical composition includes a mixture of an amount of one or more compounds selected from vitamin D₃, 1, 25-dihydroxyvitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10 (19)-triene, and pharmaceutically acceptable salts thereof, which is effective to promote synthesis of nerve growth factor when administered topically; an amount of one or more compounds selected from quercetin and pharmaceutically acceptable salts thereof, which is effective when administered topically in the composition to inhibit aldose reductase; a topically effective amount of one or more antioxidants; and a topical carrier.

### DETAILED DESCRIPTION OF THE PREFERRED EMBODIMENTS

In a first aspect, the present invention relates to a topical composition for use in treatment of diabetic neuropathy. The composition includes at least a compound that promotes synthesis of nerve growth factor, an aldose reductase inhibitor and an antioxidant.

The compound that promotes synthesis of nerve growth factor may be selected from suitable compounds that have been shown to have this activity. Suitable compounds that promote synthesis of nerve growth factor are those that do not induce significant, adverse side effects when administered to a patient in amounts that promote synthesis of nerve growth factor, and which do not react with one or more of the ingredients of the compositions of the invention thereby resulting in a substantial loss of activity of one or more active ingredients. Preferred compounds for promoting synthesis of nerve growth factor are those that occur naturally in the human body and/or materials obtained from plants, animals or derivatives thereof, which may be administered to humans without significant, adverse side effects in the amounts used.

The compounds that promote synthesis of nerve growth factor useful in the present invention are vitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10 (19)-triene and 1, 25-dihydroxyvitamin D₃. Also, pharmaceutically acceptable salts of these compounds, that promote synthesis of nerve growth factor may be employed. The most preferred compound that promotes synthesis of nerve growth factor is vitamin D₃.

The preferred compounds that promote the synthesis of nerve growth factor may, in addition to this activity, also function to prevent neurotrophic deficits. This additional effect of the preferred compounds may also contribute to the overall beneficial effect of the composition of the present invention.

In order to formulate the compound that promotes synthesis of nerve growth factor in the composition of the present invention, it may be necessary to use a dispersant. Suitable dispersants are known to persons skilled in the art. A particularly suitable dispersant for the compounds that promote, synthesis of nerve growth factor is corn oil. Corn oil also has, the advantage that it is a natural product. The amount of corn oil used is an amount sufficient to disperse the compound that promotes synthesis of nerve growth factor.

The second ingredient of the composition of the present invention is an aldose reductase inhibitor. Numerous suitable aldose reductase inhibitors are known to persons skilled in the art. Again, suitable aldose reductase inhibitors are those that do not induce significant, adverse side effects when administered to a patient in an amount effective for aldose reductase inhibition, and which do not react with one or more of the ingredients of the composition of the present invention thereby resulting in a substantial loss of activity of one or more active ingredients of the composition. Preferred aldose reductase inhibitors are those that occur naturally in the human body and/or materials obtained from plants, animals, or derivatives thereof, which may be administered to humans without significant, adverse side effects in the amounts used.

As mentioned above, numerous aldose reductase inhibitors are known to persons skilled in the art. However, significant adverse side effects are associated with the use of many aldose reductase inhibitors in humans. Thus, it is important to select one or more aldose reductase inhibitors for use in the compositions of the present invention based on minimizing the risk associated with use of the aldose reductase inhibitor taking into account the amount of that particular inhibitor that must be employed to achieve the desired level of aldose reductase inhibition. Different aldose reductase inhibitors exhibit different levels of inhibition. With this in mind, the aldose reductase inhibitor for use in the compositions of the present invention is quercetin, since this compound exhibits a high level of aldose reductase inhibition in combination with a relatively low toxicity. Also, pharmaceutically acceptable salts of this aldose reductase inhibitor may be employed.

Another active ingredient in the composition of the present invention is the antioxidant. The antioxidant may be a single compound or a mixture of two or more compounds. Also, the antioxidant may include one or more compounds that provide additional beneficial effects beyond the antioxidant activity, such as aldose reductase inhibition.

Compounds which may be used as antioxidants are those which exhibit antioxidant activity when administered without causing any severe adverse side affects when used in an amount effective to provide sufficient antioxidant activity, and which do not react with one or more of the ingredients of the composition of the present invention thereby resulting in a substantial loss of activity of one or more active ingredients. Preferred antioxidants are those that occur naturally in the human body and/or materials obtained from plants or animals which may be administered to humans without significant, adverse side effects in the amounts used, or derivatives thereof

Preferred antioxidants are selected from ascorbyl palmitat, ascorbic acid (vitamin C), vitamin A, vitamin E acetate, α-lipoic acid, especially DL- α-lipoic acid, coenzyme Q10, glutathione, (-)-epigallocatechin-3-gallate, catechin, galangin, rutin, luteolin, morin, fisetin, silymarin, apigenin, gingkolides, hesperitin, cyanidin, citrin, curcuminoid and derivatives thereof which exhibit antioxidant activity.

Even more preferably, mixtures of two or more antioxidants are employed in the composition of the present invention. Particularly preferred antioxidant mixtures are mixtures of ascorbyl palmitate with one or more of vitamin A, vitamin E acetate and α-lipoic acid, especially DL-α-lipoic acid. Derivatives of one or more of these compounds, which exhibit antioxidant activity when administered in the compositions of the present invention may also be employed. The antioxidants may also be used in the form of their pharmaceutically acceptable salts and this may be preferred in some cases to increase solubility or dispersability, to reduce adverse side effects, etc.

By "derivatives" is meant structurally similar compounds that exhibit antioxidant activity and contain at least one significant, common structural element with the compound from which it is derived, which common structural element provides antioxidant activity.

In another preferred embodiment, the antioxidant used in the composition of the present invention may be partially or completely replaced with an amount of one or more antioxidant enzymes having a comparable level of activity. The antioxidant enzymes useful in the present invention are those capable of scavenging radicals, promoting radical scavengers or preventing radical formation. In one more preferred embodiment, the antioxidant enzyme used in the present invention is skin absorbable. The preferred antioxidant enzymes for use in the present invention include superoxide dismutase, catalase, glutathione peroxidase, methionine reductase and equivalents thereof. These antioxidant enzymes may prevent the formation of free radicals or scavenge the formed free radicals to prevent cell damage. In addition, one or more of these antioxidant enzymes may act synergistically with one or more of the antioxidants in the composition to scavenge free radicals more effectively and thereby help to prevent cell damage in the skin.

The antioxidants used in the composition of the present invention are preferably selected not only for their antioxidant activity, but also based on other beneficial effects that particular compounds may provide. For example, ascorbyl palmitate not only has antioxidant activity, but also may act as an aldose reductase inhibitor and may help prevent degradation of nitric oxide (NO) and thus is a particularly preferred antioxidant for the present invention. Similarly, vitamin E may also help prevent degradation of nitric oxide and is thus a preferred antioxidant. A racemic mixture of α-lipoic acid not only has a strong antioxidant activity but also has a recycling effect on vitamins C and E, and thus is a particularly preferred antioxidant for the present invention. In addition, α-lipoic acid can function in both lipid and non-lipid environments. Similarly, vitamin E may also contribute to an anticancer effect and provide a further beneficial effect on the skin and is thus a preferred antioxidant in one embodiment of the present invention. Vitamin A (retinol or retinyl ester) may also have anticancer effects. Vitamin A is also a fat-soluble material and thus is preferred for use due to this additional beneficial property. However, due to its solubility characteristics, vitamin A may need to be formulated in a suitable dispersant such as corn oil in much the same manner as vitamin D₃ as described above.

Preferably, the antioxidant used in the composition of the present invention includes a combination of effective amounts of vitamin A, vitamin, C or its ester, vitamin E and α-lipoic acid to achieve, in addition to the antioxidant effect, the beneficial effect of recycling vitamin C or its ester and vitamin E by α-lipoic acid.

In another preferred embodiment, antioxidant mixtures including ascorbyl palmitate with one or both of vitamin A and vitamin E as tocopherols or vitamin E as mixed tocopherols are employed in the composition of the present invention. Most preferably, all-natural vitamin E tocopherols are employed. The antioxidants may also be used in the form of their pharmaceutically acceptable salts and this may be preferred in some cases to increase solubility or dispersability, to reduce adverse side effects, to increase bioavailability, etc.

In a more preferred embodiment, both quercetin and ascorbyl palmitate are included in the composition of the present invention because there seems to be an enhanced antioxidative effect of the combination of quercetin and ascorbyl palmitate.

The antioxidant component of the composition is used in an amount effective to provide significant antioxidant activity when administered to a patient in the composition of the present invention.

Preferably, vitamins A and D₃ may be formulated in a single corn oil dispersion. Generally, each cubic centimeter (cc) of the corn oil dispersion of vitamins A and D₃ used in the present invention may contain 500,000 to 2,000,000 IU of vitamin A and 50,000 to 200,000 IU of vitamin D₃. Preferably, every cc of the corn oil dispersion of vitamins A and D₃ used in the present invention may contain 800,000 to 1,200,000 IU of vitamin A and 80,000 IU to 120,000 IU of vitamin D₃. The most preferred amounts of vitamins A and D₃ employed in the composition of the invention are 1,000,000 IU and 100,000 IU, respectively.

The aldose reductase inhibitor is used in an amount of 0.04 to 40 grams, per gram of the antioxidant in the composition. More preferably, the aldose reductase inhibitor is employed in an amount of 0.17 to 15 grams and, most preferably, 0.4 to 4 grams of the aldose reductase inhibitor; per gram of the total antioxidant in the composition, is employed. The ratio of the amount of the compound that promotes synthesis of nerve growth factor to the amount of antioxidant employed in the compositions of the present invention is from 200 IU per gram of antioxidant to 3 million IU, per gram of antioxidant. More preferably, 1800 IU to 1 million IU, per gram of antioxidant, and, most preferably 5000 IU to 200,000 IU, per gram of antioxidant, of the compound that promotes synthesis of nerve growth factor is employed.

The compositions of the present invention may provide one or more of the following beneficial effects to a patient when administered in effective amounts: relief of pain, burning, tingling, electrical sensations and/or hyperalgesia, increased microcirculation, nitric oxide stabilization, promotion of healing of skin ulcers and lesions, protein kinase C inhibition, decreased oxidative stress, anti-inflammation, blockage of the formation of leukotrienes, stabilization of cell membranes, and promotion of the synthesis of nerve growth factor.

Other compounds may also be included in the composition of the present invention to provide additional benefits. Desirable additional beneficial properties for the other compounds useful in the composition of the present invention include absorbability when applied topically, aldose reductase inhibition, antioxidant properties, free radical scavenging, transition metal chelation, nitric oxide stabilization, and anti-inflammatory activity, which may have a beneficial effect on the pain of other related disorders such as fibromyalgia.

In a second aspect, the present invention provides a composition for use in the treatment of diabetic neuropathy involving the administration of a composition of the present invention by topical administration. The composition is to be administered to topically to a patient that suffers from diabetic neuropathy. The composition of the invention is to be administered one to six times daily as needed to relieve pain and other symptoms of the diabetic neuropathy.

The present invention involves a composition to be administered topically to an area of the skin in the vicinity of tissue that suffers from diabetic neuropathy. In particular, the present invention is useful on the patients' extremities such as the fingers, toes, hands and feet where diabetic neuropathy is often the most pervasive.

In the topical embodiment, the topical composition preferably includes a pharmaceutically acceptable topical carrier. In the method, a suitable amount of the topical composition of the invention is applied one to six times daily as needed to an area of the skin in the vicinity of tissue that suffers from diabetic neuropathy in order to relieve pain and other symptoms of the diabetic neuropathy. Preferably, the topical composition is applied two to four times daily, as needed for pain. In the preferred method, a sufficient amount of the topical composition is applied to cover the afflicted area with a thin layer of the composition and the composition is rubbed into the skin until little or no residue remains on the skin. Treatment initially addresses acute symptoms but may be continued, indefinitely to relieve pain, prevent symptoms of diabetic neuropathy from returning and possibly restore some nerve and/or skin function.

The topical embodiment of the method of the present invention may provide one or more of the beneficial effects described above for the composition of the invention. In addition, the method of the present invention employing the topical composition may provide some additional beneficial effects due to one or more of the ingredients contained in the pharmaceutically acceptable topical carrier as described in more detail below.

The pharmaceutically acceptable topical carrier of the present invention is suitable for use as a carrier for a topical composition wherein the active ingredients are dissolved, dispersed and/or suspended in the composition. The topical carrier of the present invention contains at least a hydrophilic ointment base, panthenol or a panthenol derivative and one or more dispersants, if needed to disperse one or more insoluble or partially insoluble active agents in the carrier. Another preferred carrier of the present invention employs hydroxymethyl cellulose as the carrier material.

Yet another preferred pharmaceutically acceptable carrier may include a solution of an acrylic copolymer in a non-aqueous solvent system, which mainly contains polyethylene glycol such as methoxy polyethylene glycol 550 (MPEG). A particular preferred MPEG is SENTRY CARBOWAX MPEG 550 sold by Union Carbide, which is a food/pharmaceutical/cosmetic grade material. Polyethylene glycols are generally nontoxic, water-soluble polymers that are fully biodegradable. In the solution, the acrylic copolymer would preferably be present in a concentration range of 3-6 % by weight. Preferably, the acrylic copolymer has a molecular weight of more than 20,000. More preferably, the acrylic copolymer has a molecular weight of more than 100,000, so that the acrylic copolymer will not be substantially absorbed by the human body through the skin.

Suitable hydrophilic ointment bases are known to persons skilled in the art. Exemplary hydrophilic ointment bases suitable for use in the present invention are non-U.S.P. hydrophilic ointment bases such as those made by Fougera, Inc. Sufficient hydrophilic ointment base is employed to act as a carrier for the active ingredients of the composition. Typically the hydrophilic ointment base will make up more than 80% of the total composition and more preferably 80-90% of the composition is the hydrophilic ointment base. The hydrophilic ointment base functions as a carrier and preferably enhances penetration of the active agents into the skin.

The panthenol or panthenol derivatives useful in the present invention include at least D-panthenol, DL-panthenol and mixtures thereof. This component of the topical carrier has skin moisturizing properties, acts as a quick, deep penetrating component of the carrier that helps deliver the active agents through the skin to the area to be treated and imparts a healing effect to damaged tissue. The amount of panthenol or panthenol derivative to be employed is from 0.25 to 10 weight percent, more preferably, from 0.5 to 5 weight percent, and, most preferably, from 1 to 2 weight percent is employed, based on the total weight of the topical composition.

The topical carrier of the present invention may also include additional ingredients known to persons skilled in the art such as other carrier materials, moisturizers, humectants, emollients dispersants, radiation blocking compounds, particularly UV-blockers, as well as other suitable materials that do not have a significant adverse effect on the activity of the topical composition in the amount used. Preferred additional ingredients for inclusion in the carrier are sodium acid phosphate as a moisturizer, witch hazel extract as a carrier, glycerine as a humectant, apricot kernal oil as an emollient, and corn oil as a dispersant.

Other materials that may optionally be included in the topical composition of the present invention include inositol, other B-complex vitamins, and anti-inflammatory agents. The composition of the present invention may also be employed to facilitate wound healing, for the treatment of skin cancer and/or one or more symptoms thereof, or as a composition for protecting skin from the harmful effects of radiation such as radiation breakdown.

The topical composition of the present invention is preferably made by cold compounding. This may be an important feature of the invention since one or more of the compounds employed in the topical composition are known to be sensitive to heat or other types of energy and thus the activity of the composition may be detrimentally affected as a result of formulating the composition in other ways. Thus, the ingredients of the topical composition the present invention are preferably mixed together, without heating, using a sufficient amount of the topical carrier to provide a substantially homogeneous cream or ointment. It may be necessary to dissolve, disperse or suspend one or more of the ingredients prior to cold compounding in order to ensure substantially homogeneous distribution of one or more of the ingredients in the composition.

Every kilogram of a preferred topical composition of the present invention may include: 10,000 to 3 million IU of the compound that promotes synthesis of nerve growth factor, 2 to 40 grams of aldose reductase inhibitor, 1 to 50 grams of antioxidants, and other suitable ingredients such as topical carriers.

A more preferred topical composition of the invention can be made using the following ingredients, all based on use of one pound of hydrophilic ointment base. 25-35 cc of a 50% aqueous solution of sodium acid phosphate moisturizing agent, 5-10 cc of D-or DL-panthenol, 5-10 cc of glycerine, 1-3 cc of apricot kernal oil, 3-5 cc of a dispersion of vitamins A and D₃ in a corn oil base, 10-20 cc of witch hazel extract, 0.5-2 cc of vitamin E acetate, 2-4 grams of ascorbyl palmitate and 4-8 grams of quercetin powder. Optionally, one or more of the glycerin, witch hazel extract, vitamins A and E and/or the ascorbyl palmitate can be reduced or eliminated from a particular composition, if desirable, or larger amounts of one or more components, i.e. antioxidant, can be employed while reducing the amount of another component of the same type or having a similar type of activity.

The invention will now be further illustrated by the following example.

### EXAMPLE 1

A topical composition including a mixture of an hydrophilic ointment base, sodium acid phosphate moisturizing agent, witch hazel extract, glycerine, apricot kernal oil and DL-panthenol, formulated together as the pharmaceutically acceptable carrier, and further including, as active agents, vitamins A and D₃, ascorbyl palmitate, quercetin and vitamin E acetate, was prepared by cold compounding. The formulation of the composition is given in Table 1.

The composition was prepared by first placing the hydrophilic ointment base in a stainless steel bowl and mixing briskly until the ointment becomes creamy. Then, the sodium acid phosphate, panthenol, ascorbyl palmitate, glycerine, apricot kernal oil, vitamins A and D₃, witch hazel extract, vitamin E acetate and quercetin are added in that order. After each ingredient is added, mixing is continued until all traces of dry ingredients have disappeared and a substantially homogeneous mixture is obtained. The final color should be a consistent yellow and the cream should have the consistency of cake frosting. The mixture is then placed in a sterile container. All containers, which contact the composition during mixing must also be sterilized with, for example, zephiran choride or a chlorox solution such as betadine.

This composition was topically administered, under the supervision of a physician, to several patients diagnosed with the most difficult cases of diabetic peripheral neuropathy. The topical composition was applied twice daily in the morning and afternoon, except that patients were permitted to apply the composition up to six times daily, as needed for pain relief over a period of a few days. All of the eight patients treated experienced immediate positive results that lasted up to a day or two after treatment was discontinued. The effects noted by the patients included the relief of burning pain, tingling, healing of damaged skin, and reversal of skin discoloration due to impaired circulation.

**Table 1**

| Ingredient | Amount Employed |
|---|---|
| Hydrophilic ointment base | 1 pound |
| 50% aqueous solution of Sodium acid phosphate | 25 cc |
| DL-panthenol | 5 cc |
| Glycerine | 5 cc |
| Apricot kernal oil | 3 cc |
| Witch hazel extract | 12 cc |
| Vitamin A and D₃ dispersion in corn oil | 4 cc |
| Vitamin E acetate | 1 cc |
| Ascorbyl Palmitate | 2 grams |
| Quercetin powder | 4 grams |

## Claims

1. A composition for use in the treatment of diabetic neuropathy including a mixture of an amount of one or more compounds selected from vitamin D₃, 1,25-dihydroxyvitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10 (19)-triene, and pharmaceutically acceptable salts thereof, which is effective to promote synthesis of nerve growth factor when administered topically; an amount of one or more compounds selected from quercetin and pharmaceutically acceptable salts thereof, which is effective when administered topically in the composition to inhibit aldose reductase; a topically effective amount of one or more antioxidants; and a topical carrier, wherein said composition is to be administered topically.

2. The topical composition for use as claimed in claim 1, wherein the one or more compounds that promote synthesis of nerve growth factor includes a compound selected from vitamin D₃ and pharmaceutically acceptable salts thereof.

3. The topical composition for use as claimed in any one of claims 1-2, wherein the one or more antioxidants are selected from: ascorbyl palmitate, ascorbic acid, vitamin A, vitamin E acetate, α-lipoic acid, coenzyme Q10, glutathione, (-)-epigallocatechin-3-gallate, catechin, galangin, rutin, luteolin, morin, fisetin, silymarin, apigenin, gingkolides, hesperitin, cyanidin, citrin, curcuminoid, and pharmaceutically acceptable salts thereof.

4. The topical composition for use as claimed in any one of claims 1-3, wherein the one or more antioxidants include one or more antioxidant enzymes.

5. The topical composition for use as claimed in any one of claims 1-4, wherein the aldose reductase inhibitor includes quercetin.

6. The topical composition for use as claimed in any one of claims 1-5, wherein the topical composition includes 2 to 40 grams of aldose reductase inhibitor per kilogram of the composition.

7. The topical composition for use as claimed in any one of claims 1-6, wherein the topical composition includes 10,000 to 3 million International Units of the compound which promotes synthesis of nerve growth factor per kilogram of the topical composition.

8. The topical composition for use as claimed in any one of claims 1-7, wherein the topical composition includes 1 to 50 grams of the one or more antioxidants per kilogram of the topical composition.

9. The topical composition for use as claimed in any one of claims 1-8, wherein the topical carrier is a hydrophilic ointment base and the hydrophilic ointment base comprises 80-90% by weight of the topical composition.

10. The topical composition for use as claimed in any one of claims 1-9, wherein the one or more antioxidants include a mixture of at least two different compounds.

11. The topical composition for use as claimed in any one of claims 1-10, wherein the one or more antioxidants include vitamin E acetate.

12. The topical composition for use as claimed in any one of claims 1-11, wherein the one or more antioxidants include vitamin A.

13. The topical composition for use as claimed in any one of claims 1-12, wherein the one or more antioxidants include ascorbyl palmitate.

14. The topical composition for use as claimed in any one of claims 1-13, wherein the one or more compounds that promote synthesis of nerve growth factor include vitamin D₃.

15. The topical compositionfor use as claimed in any one of claims 1-14, wherein the topical carrier includes a sufficient amount of a panthenol selected from D-panthenol and DL-panthenol to promote penetration of one or more compounds of the composition into the skin.

16. The topical composition for use as claimed in any one of claims 1-15, made by cold compounding.

17. Use of a topical composition which includes a mixture of an effective amount of one or more compounds selected from vitamin D₃, 1, 25-dihydroxyvitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10 (19)-triene, and pharmaceutically acceptable salts thereof to promote synthesis of nerve growth factor when topically administered; an amount of one or more compounds selected from quercetin and pharmaceutically acceptable salts thereof; which is effective, when topically administered, to inhibit aldose reductase; a topically effective amount of one or more antioxidants and a topical carrier, for the manufacture of a topical medicament for topical treatment of diabetic neuropathy.

18. The use as claimed in claim 17, wherein the one or more compounds that promote synthesis of nerve growth factor are selected from: vitamin D₃ and pharmaceutically acceptable salts thereof.

19. Use as claimed in any one of claims 17-18, wherein the one or more antioxidants are selected from: ascorbyl palmitate, ascorbic acid, vitamin A, vitamin E acetate, α-lipoic acid, coenzyrne Q10, glutathione, (-)-epigallocatechin-3-gallate, catechin, galangin, rutin, luteolin, morin, fisetin, silymarin, apigenin, gingkolides, hesperitin, cyanidin, citrin, curcuminoid, and structurally similar derivatives thereof which exhibit antioxidant activity, and pharmaceutically acceptable salts thereof.

20. The use as claimed in any one of claims 17-19, wherein the one or more antioxidants include one or more antioxidant enzymes.

21. The use as claimed in any one of claims 17-20, wherein the one or more aldose reductase inhibitors include quercetin.

22. The use as claimed in any one of claims 17-21, wherein the topical composition includes 2 to 40 grams of aldose reductase inhibitor per kilogram of the composition.

23. The use as claimed in any one of claims 17-22, wherein the topical composition includes 10,000 to 3 million International Units of the compound which promotes synthesis of nerve growth factor per kilogram of the topical composition.

24. The use as claimed in any one of claims 17-23, wherein the topical composition includes 1 to 50 grams of the one or more antioxidants per kilogram of the topical composition.

25. The use as claimed in any one of claims 17-24, wherein the topical carrier is a hydrophilic ointment base and the hydrophilic ointment base comprises 80-90% by weight of the topical composition.

26. The use as claimed in any one of claims 17-25, wherein the one or more antioxidants include a mixture of at least two different compounds.

27. The use as claimed in any one of claims 17-26, wherein the one or more antioxidants include vitamin E acetate.

28. The use as claimed in any one of claims 17-27, wherein the one or more antioxidants include vitamin A.

29. The use as claimed in any one of claims 17-28, wherein the one or more antioxidants include ascorbyl palmitate.

30. The use as claimed in any one of claims 17-29, wherein the one or more compounds that promote the synthesis of nerve growth factor include vitamin D₃.

31. The use as claimed in any one of claims 17-30, wherein the topical carrier includes a sufficient amount of a panthenol selected from D-panthenol and DL-panthenol to promote penetration of the composition into skin.

32. The use as claimed in any one of claims 17-31, wherein the topical composition is made by cold compounding.

## Patentansprüche

1. Eine Zusammensetzung für die Verwendung bei der Behandlung diabetischer Neuropathie einschließlich einer Mischung aus einer Menge einer oder mehrerer Verbindungen ausgewählt unter Vitamin D₃, 1,25-Dihydroxyvitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10(19)-trien und pharmazeutisch akzeptablen Salzen davon, welche bei topischer Verabreichung wirksam eine Synthese von Nervenwachstumsfaktor fördert; eine Menge einer oder mehrerer Verbindungen ausgewählt unter Quercetin und pharmazeutisch akzeptablen Salzen davon, welche bei topischer Verabreichung in der Zusammensetzung wirksam Aldosereduktase hemmt; eine topisch wirksame Menge eines oder mehrerer Antioxidantien; und ein topischer Träger, wobei die besagte Zusammensetzung topisch zu verabreichen ist.

2. Die topische Zusammensetzung für die Verwendung gemäß Anspruch 1, wobei die eine oder die mehreren Verbindungen, welche eine Synthese von Nervenwachstumsfaktor fördern, eine oder mehrere Verbindungen umfassen, welche unter Vitamin D₃ und pharmazeutisch akzeptablen Salzen davon ausgewählt werden.

3. Die topische Zusammensetzung für die Verwendung gemäß irgendeinem der Ansprüche 1-2, wobei das eine oder die mehreren Antioxidantien ausgewählt werden unter: Ascorbylpalmitat, Ascorbinsäure, Vitamin A, Vitamin-E-Acetat, α-Liponsäure, Coenzym Q10, Glutathion, (-)-Epigallocatechin-3-gallat, Catechin, Galangin, Rutin, Luteolin, Morin, Fisetin, Silymarin, Apigenin, Gingkolide, Hesperitin, Cyanidin, Citrin, Curcuminoid und pharmazeutisch akzeptablen Salzen davon.

4. Die topische Zusammensetzung für die Verwendung gemäß irgendeinem der Ansprüche 1-3, wobei das eine oder die mehreren Antioxidantien ein oder mehrere Antioxidans-Enzyme umfassen.

5. Die topische Zusammensetzung für die Verwendung gemäß irgendeinem der Ansprüche 1-4, wobei der Aldosereduktasehemmer Quercetin umfasst.

6. Die topische Zusammensetzung für die Verwendung gemäß irgendeinem der Ansprüche 1-5, wobei die topische Zusammensetzung 2 bis 40 Gramm Aldosereduktasehemmer pro Kilogramm der Zusammensetzung umfasst.

7. Die topische Zusammensetzung für die Verwendung gemäß irgendeinem der Ansprüche 1-6, wobei die topische Zusammensetzung 10.000 bis 3 Millionen Internationale Einheiten der Verbindung, welche eine Synthese von Nervenwachstumsfaktor fördert, pro Kilogramm der topischen Zusammensetzung umfasst.

8. Die topische Zusammensetzung für die Verwendung gemäß irgendeinem der Ansprüche 1-7, wobei die topische Zusammensetzung 1 bis 50 Gramm des einen oder der mehreren Antioxidantien pro Kilogramm der topischen Zusammensetzung umfasst.

9. Die topische Zusammensetzung für die Verwendung gemäß irgendeinem der Ansprüche 1-8, wobei der topische Träger eine hydrophile Salbengrundlage ist und die hydrophile Salbengrundlage 80-90 Gewichtsprozent der topischen Zusammensetzung beinhaltet.

10. Die topische Zusammensetzung für die Verwendung gemäß irgendeinem der Ansprüche 1-9, wobei das eine oder die mehreren Antioxidantien eine Mischung aus mindestens zwei verschiedenen Verbindungen umfassen.

11. Die topische Zusammensetzung für die Verwendung gemäß irgendeinem der Ansprüche 1-10, wobei das eine oder die mehreren Antioxidantien Vitamin-E-Acetat umfassen.

12. Die topische Zusammensetzung für die Verwendung gemäß irgendeinem der Ansprüche 1-11, wobei das eine oder die mehreren Antioxidantien Vitamin A umfassen.

13. Die topische Zusammensetzung für die Verwendung gemäß irgendeinem der Ansprüche 1-12, wobei das eine oder die mehreren Antioxidantien Ascorbylpalmitat umfassen.

14. Die topische Zusammensetzung für die Verwendung gemäß irgendeinem der Ansprüche 1-13, wobei die eine oder die mehreren Verbindungen, welche eine Synthese von Nervenwachstumsfaktor fördern, Vitamin D₃ umfassen.

15. Die topische Zusammensetzung für die Verwendung gemäß irgendeinem der Ansprüche 1-14, wobei der topische Träger eine ausreichende Menge eines unter D-Panthenol und DL-Panthenol ausgewählten Panthenols umfasst, um die Penetration der einen oder der mehreren Verbindungen der Zusammensetzung in die Haut zu fördern.

16. Die topische Zusammensetzung für die Verwendung gemäß irgendeinem der Ansprüche 1-15, durch Kaltmischen hergestellt.

17. Verwendung einer topischen Zusammensetzung, welche eine Mischung aus einer wirksamen Menge einer oder mehrerer Verbindungen umfasst, ausgewählt unter Vitamin D₃, 1,25-Dihydroxyvitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10(19)-trien und pharmazeutisch akzeptablen Salzen davon, um bei topischer Verabreichung eine Synthese von Nervenwachstumsfaktor zu fördern; eine Menge einer oder mehrerer Verbindungen ausgewählt unter Quercetin und pharmazeutisch akzeptablen Salzen davon; welche bei topischer Verabreichung wirksam Aldosereduktase hemmt; eine topisch wirksame Menge eines oder mehrerer Antioxidantien und ein topischer Träger, für die Herstellung eines topischen Arzneimittels zur topischen Behandlung diabetischer Neuropathie.

18. Die Verwendung gemäß Anspruch 17, wobei die eine oder die mehreren Verbindungen, welche eine Synthese von Nervenwachstumsfaktor fördern, ausgewählt werden unter: Vitamin D₃ und pharmazeutisch akzeptablen Salzen davon.

19. Die Verwendung gemäß irgendeinem der Ansprüche 17-18, wobei das eine oder die mehreren Antioxidantien ausgewählt werden unter: Ascorbylpalmitat, Ascorbinsäure, Vitamin A, Vitamin-E-Acetat, α-Liponsäure, Coenzym Q10, Glutathion, (-)-Epigallocatechin-3-gallat, Catechin, Galangin, Rutin, Luteolin, Morin, Fisetin, Silymarin, Apigenin, Gingkolide, Hesperitin, Cyanidin, Citrin, Curcuminoid und strukturell ähnlichen Derivaten davon, welche Antioxidationsaktivität zeigen, sowie pharmazeutisch akzeptablen Salzen davon.

20. Die Verwendung gemäß irgendeinem der Ansprüche 17-19, wobei das eine oder die mehreren Antioxidantien ein oder mehrere Antioxidans-Enzyme umfassen.

21. Die Verwendung gemäß irgendeinem der Ansprüche 17-20, wobei der eine oder die mehreren Aldosereduktasehemmer Quercetin umfassen.

22. Die Verwendung gemäß irgendeinem der Ansprüche 17-21, wobei die topische Zusammensetzung 2 bis 40 Gramm Aldosereduktasehemmer pro Kilogramm der Zusammensetzung umfasst.

23. Die Verwendung gemäß irgendeinem der Ansprüche 17-22, wobei die topische Zusammensetzung 10.000 bis 3 Millionen Internationale Einheiten der Verbindung, welche eine Synthese von Nervenwachstumsfaktor fördert, pro Kilogramm der topischen Zusammensetzung umfasst.

24. Die Verwendung gemäß irgendeinem der Ansprüche 17-23, wobei die topische Zusammensetzung 1 bis 50 Gramm des einen oder der mehreren Antioxidantien pro Kilogramm der topischen Zusammensetzung umfasst.

25. Die Verwendung gemäß irgendeinem der Ansprüche 17-24, wobei der topische Träger eine hydrophile Salbengrundlage ist und die hydrophile Salbengrundlage 80-90 Gewichtsprozent der topischen Zusammensetzung beinhaltet.

26. Die Verwendung gemäß irgendeinem der Ansprüche 17-25, wobei das eine oder die mehreren Antioxidantien eine Mischung aus mindestens zwei verschiedenen Verbindungen umfassen.

27. Die Verwendung gemäß irgendeinem der Ansprüche 17-26, wobei das eine oder die mehreren Antioxidantien Vitamin-E-Acetat umfassen.

28. Die Verwendung gemäß irgendeinem der Ansprüche 17-27, wobei das eine oder die mehreren Antioxidantien Vitamin A umfassen.

29. Die Verwendung gemäß irgendeinem der Ansprüche 17-28, wobei das eine oder die mehreren Antioxidantien Ascorbylpalmitat umfassen.

30. Die Verwendung gemäß irgendeinem der Ansprüche 17-29, wobei die eine oder die mehreren Verbindungen, welche die Synthese von Nervenwachstumsfaktor fördern, Vitamin D₃ umfassen.

31. Die Verwendung gemäß irgendeinem der Ansprüche 17-30, wobei der topische Träger eine ausreichende Menge eines unter D-Panthenol und DL-Panthenol ausgewählten Panthenols umfasst, um die Penetration der Zusammensetzung in die Haut zu fördern.

32. Die Verwendung gemäß irgendeinem der Ansprüche 17-31, wobei die topische Zusammensetzung durch Kaltmischen hergestellt wird.

## Revendications

1. Une composition destinée à être utilisée dans le traitement de la neuropathie diabétique comprenant un mélange d'une quantité d'un ou plusieurs composés choisis parmi la vitamine D₃, 1, 25-dihydroxyvitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10 (19)-triene, et leurs sels pharmaceutiquement acceptables, qui est efficace pour favoriser la synthèse du facteur de croissance des nerfs lorsqu'elle est administrée de façon topique ; une quantité d'un ou plusieurs composés choisis parmi la quercétine et ses sels pharmaceutiquement acceptables, qui est efficace lorsqu'elle est administrée de façon topique dans la composition afin d'inhiber l'aldose réductase ; une quantité d'un ou plusieurs antioxydants efficace de façon topique ; et un excipient topique, dans laquelle ladite composition doit être administrée de façon topique.

2. La composition topique à utiliser selon la revendication 1 dans laquelle un ou plusieurs composés qui favorisent la synthèse du facteur de la croissance des nerfs comprennent un composé choisi parmi la vitamine D₃ et ses sels pharmaceutiquement acceptables.

3. La composition topique à utiliser selon l'une quelconque des revendications 1 et 2 dans laquelle un ou plusieurs antioxydants sont choisis parmi : le palmitate d'ascorbyle, l'acide ascorbique, la vitamine A, la vitamine E acétate, l'acide α-lipoïque, la coenzyme Q10, le glutathion, l'(-)-epigallocatechin-3-gallate, la catéchine, la galangine, la rutine, la lutéoléine, la morine, la fustine, le silymarin, l'apigénine, les gingkolides, l'hespéritine, la cyanidine, la citrine, un curcuminoïde et des sels pharmaceutiquement acceptables de ceux-ci.

4. La composition topique à utiliser selon l'une quelconque des revendications 1-3 dans laquelle un ou plusieurs antioxydants comprennent un ou plusieurs enzymes antioxydantes.

5. La composition topique à utiliser selon l'une quelconque des revendications 1-4 dans laquelle l'inhibiteur d'aldose réductase comprend de la quercétine.

6. La composition topique à utiliser selon l'une quelconque des revendications 1-5 dans laquelle la composition topique comprend entre 2 et 40 grammes d'inhibiteur d'aldose réductase par kilogramme de composition.

7. La composition topique à utiliser selon l'une quelconque des revendications 1-6 dans laquelle la composition topique comprend entre 10 000 et 3 millions d'unités internationales du composé qui favorise la synthèse du facteur de croissance des nerfs par kilogramme de la composition topique.

8. La composition topique à utiliser selon l'une quelconque des revendications 1-7 dans laquelle la composition topique comprend entre 1 et 50 grammes d'un ou plusieurs antioxydants par kilogrammes de la composition topique.

9. La composition topique à utiliser selon l'une quelconque des revendications 1-8 dans laquelle l'excipient topique est un excipient émulsionné et l'excipient émulsionné comprend de 80 à 90 % par poids de la composition topique.

10. La composition topique à utiliser selon l'une quelconque des revendications 1-9 dans laquelle un ou plusieurs antioxydants comprennent un mélange d'au moins deux composés différents.

11. La composition topique à utiliser selon l'une quelconque des revendications 1-10 dans laquelle un ou plusieurs antioxydants comprennent de la vitamine E acétate.

12. La composition topique à utiliser selon l'une quelconque des revendications 1-11 dans laquelle un ou plusieurs antioxydants comprennent de la vitamine A.

13. La composition topique à utiliser selon l'une quelconque des revendications 1-12 dans laquelle un ou plusieurs antioxydants comprennent du palmitate d'ascorbyle.

14. La composition topique à utiliser selon l'une quelconque des revendications 1-13 dans laquelle un ou plusieurs composés qui favorisent la synthèse du facteur de croissance des nerfs comprennent de la vitamine D₃.

15. La composition topique à utiliser selon l'une quelconque des revendications 1-14 dans laquelle l'excipient topique comprend une quantité suffisante d'un panthénol choisi parmi le D-panthénol et le DL-panthénol afin de favoriser la pénétration dans la peau d'un ou plusieurs composés de la composition.

16. La composition topique à utiliser selon l'une quelconque des revendications 1-15, faite par mélange à froid.

17. L'utilisation d'une composition topique qui comprend un mélange d'une quantité efficace d'un ou plusieurs composés choisis parmi la vitamine D₃, 1, 25-dihydroxyvitamin D₃, 1(S), 3(R)-dihydroxy-20(R)-(1-ethoxy-5-ethyl-5-hydroxy-2-heptyn-1-yl)-9, 10-seco-pregna-5(Z), 7(E), 10 (19)-triene, et leurs sels pharmaceutiquement acceptables pour favoriser la synthèse du facteur de croissance des nerfs lorsqu'elle est administrée de façon topique ; une quantité d'un ou plusieurs composés choisis parmi la quercétine et ses sels pharmaceutiquement acceptables ; qui est efficace lorsqu'elle est administrée de façon topique pour inhiber l'aldose réductase ; une quantité d'un ou plusieurs antioxydants qui est efficace lorsqu'elle administrée de façon topique et un excipient topique, pour la fabrication d'un médicament topique pour le traitement topique de la neuropathie diabétique.

18. L'utilisation selon la revendication 17 dans laquelle un ou plusieurs composés qui favorisent la synthèse du facteur de la croissance des nerfs sont choisis parmi : la vitamine D₃ et ses sels pharmaceutiquement acceptables.

19. L'utilisation selon l'une quelconque des revendications 17 et 18 dans laquelle un ou plusieurs antioxydants sont choisis parmi : le palmitate d'ascorbyle, l'acide ascorbique, la vitamine A, la vitamine E acétate, l'acide α-lipoïque, la coenzyme Q10, le glutathion, l'(-)-epigallocatechin-3-gallate, la catéchine, la galangine, la rutine, la lutéoléine, la morine, la fustine, le silymarin, l'apigénine, les gingkolides, l'hespéritine, la cyanidine, la citrine, un curcuminoïde et leurs dérivés similaires en structure qui produisent une activité antioxydante, et leurs sels pharmaceutiquement acceptables.

20. L'utilisation selon l'une quelconque des revendications 17 -19 dans laquelle un ou plusieurs antioxydants comprennent une ou plusieurs enzymes antioxydantes.

21. L'utilisation selon l'une quelconque des revendications 17 - 20 dans laquelle un ou plusieurs inhibiteurs d'aldose réductase comprend la quercétine.

22. L'utilisation selon l'une quelconque des revendications 17 - 21 dans laquelle la composition topique comprend entre 2 et 40 grammes d'inhibiteur d'aldose réductase par kilogramme de la composition.

23. L'utilisation selon l'une quelconque des revendications 17 - 22 dans laquelle la composition topique comprend entre 10 000 et 3 millions d'unités internationales de la composition qui favorise la synthèse du facteur de croissance des nerfs par kilogramme de composition topique.

24. L'utilisation selon l'une quelconque des revendications 17 - 23 dans laquelle la composition topique comprend entre 1 et 50 grammes d'un ou plusieurs antioxydants par kilogramme de composition topique.

25. L'utilisation selon l'une quelconque des revendications 17 - 24 dans laquelle l'excipient topique est un excipient émulsionné et l'excipient émulsionné comprend de 80 à 90 % par poids de composition topique.

26. L'utilisation selon l'une quelconque des revendications 17 - 25 dans laquelle un ou plusieurs antioxydants comprennent un mélange d'au moins deux composés différents.

27. L'utilisation selon l'une quelconque des revendications 17 - 26 dans laquelle un ou plusieurs antioxydants comprennent de la vitamine E acétate.

28. L'utilisation selon l'une quelconque des revendications 17-27 dans laquelle un ou plusieurs antioxydants comprennent de la vitamine A.

29. L'utilisation selon l'une quelconque des revendications 17-28 dans laquelle un ou plusieurs antioxydants comprennent du palmitate d'ascorbyle.

30. L'utilisation selon l'une quelconque des revendications 17-29 dans laquelle un ou plusieurs composés qui favorisent la synthèse du facteur de croissance des nerfs comprennent de la vitamine D₃.

31. L'utilisation selon l'une quelconque des revendications 17-30 dans laquelle l'excipient topique comprend une quantité suffisante d'un panthénol choisi parmi le D-panthénol et le DL-panthénol afin de favoriser la pénétration de la composition dans la peau.

32. L'utilisation selon l'une quelconque des revendications 17-31 dans laquelle la composition topique est faite par mélange à froid.
